# EUROPEAN PATENT SPECIFICATION

(11) **EP 3 360 470 B1**
(45) Date of publication and mention of the grant of the patent: **10.03.2021**
(21) Application number: 16784773.0
(22) Date of filing: 04.01.2016
(51) Int. Cl.: A61B 5/021, A61B 5/022

(54) **WEARABLE BLOOD PRESSURE CUFF, SPHYGMOMANOMETER AND USAGE METHOD THEREOF**
AM KÖRPER TRAGBARE BLUTDRUCKMANSCHETTE, SPHYGMOMANOMETER UND VERWENDUNGSVERFAHREN DAFÜR
BRASSARD DE TENSIOMÈTRE PORTABLE, SPHYGMOMANOMÈTRE ET LEUR PROCÉDÉ D'UTILISATION

(30) Priority: 10.10.2015 CN 201510652056
(43) Date of publication of application: 15.08.2018
(73) Proprietor: Yang, Shengzhou, Shenzhen, Guangdong 518000 (CN)
(72) Inventor: Yang, Shengzhou, Shenzhen, Guangdong 518000 (CN)
(74) Representative: Ipside
(86) International application number: PCT/CN2016/070011
(87) International publication number: WO 2017/059650

(56) References cited:
- EP-A1- 1 669 024
- WO-A1-2007/148661
- WO-A1-2013/113333
- CN-A- 104 908 347
- CN-A- 105 078 431
- CN-U- 204 181 604
- CN-U- 204 562 272
- CN-U- 204 654 919
- JP-A- H0 564 631
- US-A1- 2012 150 051

## Description

### FIELD OF THE INVENTION

The present disclosure relates to a sphygmomanometer cuff, and more particularly to a sphygmomanometer cuff, a sphygmomanometer with the same, and a method for using the same.

### BACKGROUND OF THE INVENTION

People have paid more and more attention to their health as living standards rise. More and more families deploy a variety of home medical equipments to facilitate simple health management of their family members. Blood pressure measuring device, or sphygmomanometer, has become a necessary health monitoring equipment in people's daily lives. With the development of science and technology, sphygmomanometers have become increasingly more compact, portable, and sensitive.

Nowadays, many types of portable intelligent sphygmomanometer have been developed, such as wearable sphygmomanometers for the upper arm or the wrist. These types of sphygmomanometers require cuffs (or bands) to be wrapped and fastened around the upper arm or wrist for measuring blood pressure. The cuff is typically built-in with a fluid bag for pressing arteries at certain portions of the body (such as the upper arm or the wrist). Arterial pressure wave can be detected when air is pumped into and out of the fluid bag by a built-in pump motor, so that blood pressure can be measured. Generally, the cuff includes an air bag for accommodating air and a sleeve for wrapping the air bag around the body. When worn on the upper arm or the wrist of a user, the cuff is also called an arm band or wrist band.

In the prior art, air bags are usually of a flat one-piece sealed rubber structure. The upper and lower rubber layers of the air bag tend to adhere to each other, therefore lowering the speed of inflation and deflation of the air bag and detection efficiency of the sphygmomanometer. In addition, poor air permeability of the rubber layers reduces the comfort of measurements.

US 2012/150051 A1 discloses a blood pressure cuff, the cuff comprising: a first sheet; a second sheet, wherein a second interior-inflatable surface of the second sheet is attached to a first interior-inflatable surface of the first sheet, wherein the second interior-inflatable surface has a textured surface; and an interior-inflatable portion between the first interior-inflatable surface and the second interior-inflatable surface, wherein the interior-inflatable portion is in communication with an opening to fluidly interconnect the interior-inflatable portion with an exterior of the cuff.

### TECHNICAL PROBLEMS

To provide a solution to the aforementioned problems, an embodiment of the present invention provides a sphygmomanometer cuff with fast inflation or deflation of the air bag and comfort of the measurement, a wearable sphygmomanometer with the sphygmomanometer cuff, and a method for using the wearable sphygmomanometer.

### TECHNICAL SOLUTION

A sphygmomanometer cuff includes a cuff body for wrapping around a predetermined portion of a body. The cuff body includes an air bag, a retaining plate and an air tube. The air bag includes a top layer, a bottom layer, and an intermediate layer disposed between and spacing apart the top layer and the bottom layer. A sealed cavity is defined between the top layer and the bottom layer. The retaining plate is attached to an outer surface of the top layer of the air bag. The air tube extends through the retaining plate and connects with the sealed cavity of the air bag. An intermediate layer is disposed between the top layer and the bottom layer, and separates the top layer and the bottom layer, and the intermediate layer has a rough surface; characterized in that the retaining plate is suitable for securing a sphygmomanometer to the cuff body; and a bottom surface of the retaining plate is arcuate, the bottom surface recesses toward a top surface of the retaining plate, a plurality of securing feet are formed intermittently on a bottom of the retaining plate and intermittently contact the outer surface of the top layer of the air bag, and a plurality of interspaces are formed between any two of the securing feet at the bottom of the retaining plate and the outer surface of the top layer. A thickness of the intermediate layer may range from about 0.2 millimeters to about 2 millimeters.

Preferably, the intermediate layer is attached to an inner surface of the bottom layer, or an inner surface of the top layer.

Preferably, a bottom surface of the retaining plate is arcuate, and the bottom surface recesses toward a top surface of the retaining plate. When the wearable sphygmomanometer is worn on an arm for a long time, the retaining plate abuts against the air bag. The top layer and the bottom layer of the air bag are disposed adjacent to each other. An air channel is formed at the arcuate surface of the retaining plate between the top layer and the bottom layer and is connected with the air tube, so as to fast inflate or deflate the air bag (if a bottom surface of the retaining plate is flat, the top layer and the bottom layer are pressed tightly by the retaining plate, no air channel is defined in the top layer and the bottom layer, it is hard to inflate or deflate air into or from the air tube). In addition, a plurality of gaps is defined between the arcuate surface and the arm, so as to improve skin respiration.

A wearable sphygmomanometer includes the sphygmomanometer cuff and a sphygmomanometer main body. The sphygmomanometer main body is attached to a top surface of the retaining plate.

A method for using a wearable sphygmomanometer, comprising the following steps.

Providing a wearable sphygmomanometer.

Wrapping the sphygmomanometer cuff around the predetermined portion of the body.

When the wearable sphygmomanometer is worn on an arm for a long time, the air bag of the sphygmomanometer cuff abuts against the arm, the top layer and the bottom layer of the air bag are disposed adjacent to each other, and the rough surface of the intermediate layer of the air bag allows a plurality of gaps to form at a contact area between the cuff body and the predetermined portion of the body, so as to improve skin respiration.

When the wearable sphygmomanometer performs a measurement of blood pressure, external air flows through the air tube to inflate the sealed cavity of the air bag, and the rough surface of the intermediate layer prevents the top layer and the bottom layer from adhering to each other, so as to expedite inflation of the sealed cavity of the air bag.

When the wearable sphygmomanometer completes the measurement, air in the sealed cavity of the air bag is released through the air tube, and rough the surface of the intermediate layer prevents the top layer and the bottom layer from adhering to each other, so as to expedite deflation of the sealed cavity of the air bag.

Preferably, when the wearable sphygmomanometer is worn on an arm for a long time, the retaining plate abuts against the air bag, the top layer and the bottom layer of the air bag are disposed adjacent to each other, and an air channel is formed at the arcuate surface of the retaining plate between the top layer and the bottom layer and is connected with the air tube, so as to fast inflate or deflate the air bag.

Preferably, when the wearable sphygmomanometer is worn on an arm for a long time, the retaining plate abuts against the air bag; when the air bag is inflated, the securing feet press against the top layer, and portions of the top layer, the bottom layer, and the intermediate layer corresponding to the securing feet are compressed together in the air bag, so that the top layer and the bottom layer of the air bag abut against the plurality of securing feet to form a plurality of intermittent air passages in the sealed cavity, and the air passages are connected with the air tube, so as to fast inflate or deflate the air bag.

### BENEFICIAL EFFECT

According to the aforementioned embodiments, the present invention has the following advantages.

First, the fabric intermediate layer disposed between the top layer and the bottom layer of the air bag can prevent adhesion between the top layer and bottom layer and thus facilitate fast inflation and deflation.

Second, when the sphygmomanometer cuff is worn on the arm for a long time, the intermediate layer also allows the gaps to form between the sphygmomanometer cuff and the arm so that skin respiration and thus comfort of blood pressure measurement is enhanced.

Third, when the sphygmomanometer cuff is worn on the arm for a long time, an air channel is defined in the top layer and the bottom layer of the air bag corresponding to the arcuate surface. An air channel is defined in the arcuate surface of the retaining plate between the top layer and the bottom layer and is connected with the air tube. When the measurement is completed, air in the air bag can be quickly deflated through the air channel. The speed of deflation of the air bag is improved. Similarly, when the sphygmomanometer starts to measure blood pressure and the air bag starts to be inflated. Outer air from the air tube can fill the air bag quickly through the air channel. The speed of inflation of the air bag is improved. Thus, arcuate surface of the retaining plate can improve the measurement speed and measurement precision. In addition, when the sphygmomanometer cuff presses the arm tightly, an air gap is defined between the arcuate surface and the arm, which facilitates skin respiration.

Finally, when the sphygmomanometer cuff is worn on the arm of the user for a long time, the retaining plate abuts against the air bag, the plurality of securing feet abuts against the top layer and the bottom layer of the air bag, the top layer and the bottom layer of the air bag are attached together. A plurality of intermittent air passages is defined in the top layer and the bottom layer corresponding to gaps of the plurality of securing feet, and the air passages are connected with the air tube, so as to fast inflate or deflate the air bag through the plurality of intermittent air passages between the top layer and the bottom layer of the air bag, when the air bag is inflated or deflated. In addition, when the sphygmomanometer cuff is pressured, the air bag presses the arm tightly. The plurality of gaps is defined between the plurality of securing feet and the arm, therefore facilitating skin respiration.

### BRIEF DESCRIPTION OF THE DRAWINGS

FIG. 1 is a front view of a sphygmomanometer cuff according to one embodiment of the present invention.
FIG. 2 is a bottom view of the sphygmomanometer cuff of FIG. 1.
FIG. 3 is a state view showing an air channel being defined in the sealed cavity of the air bag when the sphygmomanometer cuff is worn, and when the retaining plate abuts against the air bag, a bottom surface of the retaining plate is arcuate.
FIG. 4 is a state view showing a plurality of intermittent air passages being defined in the sealed cavity of the air bag, when the sphygmomanometer cuff is worn, and when the retaining plate with a plurality of intermittently arranged securing feet abuts against the air bag.

### DETAILED DESCRIPTION OF PREFERRED EMBODIMENTS

In order to better understand the purpose, the embodiments and the advantages of the present invention, the following paragraphs will combine with drawings and embodiments to give detail descriptions of the present invention.

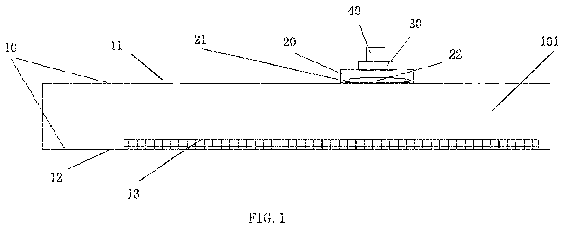

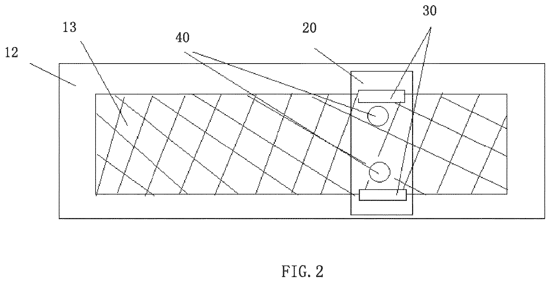

Referring to FIG. 1 and FIG. 2. In this embodiment, a sphygmomanometer cuff for arm blood pressure measurement includes a cuff body for wrapping around an upper arm or a wrist of a user. The cuff body includes an air bag 10, a retaining plate 20 for securing a sphygmomanometer to the cuff body, a fastener 30, and an air tube 40. The fastener 30 is configured to fasten the sphygmomanometer to the retaining plate 20. The air tube 40 extends through the retaining plate 20 and may secure the retaining plate 20 to the air bag 10.

In addition, the sphygmomanometer cuff may include a self-locking ring and a molding paster. The self-locking ring and the molding paster may be disposed on two opposite sides of the cuff body for fastening the sphygmomanometer cuff to the arm of the user.

The air bag 10 may be laid flat, and includes a top layer 11 and a bottom layer 12. Edges of the top layer 11 and the bottom layer 12 are adhered to form a sealed cavity 101 defined therebetween. An intermediate layer 13 is disposed between the top layer 11 and the bottom layer 12, and spaces apart the top layer 11 and the bottom layer 12. The intermediate member 13 may be made of net fabrics with rough surfaces. A thickness of the intermediate layer 13 may range from about 0.2 millimeters to about 2 millimeters. The intermediate layer 13 may be attached to the bottom layer 12 or the top layer 11; alternatively, the intermediate layer 13, the top layer 11 and the bottom layer 12 may have a substantially identical surface area so that edges of the three layers may be overlapped and adhered. It is to be understood that the present invention is not limited thereto as long as the intermediate layer 13 separates the bottom layer 12 or the top layer 11.

The air tubes 40 can be two or more. Two air tubes 40 are employed in the present embodiment. The air tubes 40 extend through the retaining plate 20 and connect with the sealed cavity of the air bag 10, so that air can enter the seal cavity during blood pressure measurement and leave the seal cavity after measurement completes.

When the sphygmomanometer starts to measure blood pressure and the air bag 10 starts to be inflated, as the top layer 11 and the bottom layer 12 are spaced apart by the intermediate layer 13, the top layer 11 and the bottom layer 12 separate quickly so that air fills the air bag 10 quickly. If no intermediate layer 13 is disposed in the air bag 10, the top layer 11 and the bottom layer 12 tend to adhere to each other, and sufficient airflow is required to break the adhesion. Thus, inflation speed of the air bag 10 and measurement speed would be reduced without adopting the intermediate layer 13.

When the measurement is completed, the air in the sealed cavity 101 begins to release. During the deflation process, as the intermediate layer 13 prevents the top layer 11 and the bottom layer 12 from adhering to each other, the air in all areas of the sealed cavity 101 can be released quickly through the air tube 40, thus increasing the speed of deflation and enhancing the comfort of measurement.

When the sphygmomanometer cuff is used on wearable sphygmomanometer, the sphygmomanometer cuff is worn on the arm of the user for a long time. When the sphygmomanometer cuff is pressured, the air bag 10 presses the arm tightly. The rough surface of the intermediate layer 13 allows a plurality of gaps to form at the contact area between the sphygmomanometer cuff and the arm, therefore facilitating skin respiration. In the present embodiment, the intermediate layer 13 is attached to and is integrated with an inner surface of the bottom layer 12. When the air bag 10 is inflated, the top layer 11 and the bottom layer 12 are separated, the bottom layer 12 of the air bag 10 would press against the arm of the user, and the plurality of gaps are formed between the sphygmomanometer cuff and the arm. Skin of the arm may respire and comfort of the measurement is enhanced.

The retaining plate 20 may be a rectangular sheet, with a length of the retaining plate 20 slightly smaller than a width of the sphygmomanometer cuff. A longitudinal direction of the retaining plate 20 is substantially parallel to a width direction of the sphygmomanometer cuff. The retaining plate 20 includes an arcuate surface 21 (for example, a half-hole disposed on the bottom surface 21 along the longitudinal direction of the retaining plate 20). The arcuate surface 21 recesses toward a top surface of the retaining plate 20. Therefore, when an outer edge of the retaining plate 20 is attached to an outer surface of the top layer 11 of the air bag 10, an air gap 22 is formed between the bottom surface 21 of the retaining plate 20 and the outer surface of the top layer 11 of the air bag 10.

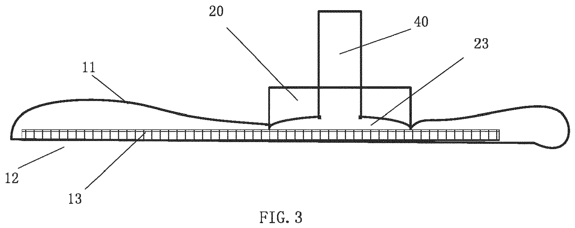

Referring to FIG. 3. When the sphygmomanometer cuff is worn on the arm of the user for a long time, the retaining plate 20 abuts against the air bag 10, and the top layer 11 and the bottom layer 12 are disposed adjacent to each other. The arcuate surface of the retaining plate 20 allows an air channel 23 to form between the top layer 11 and the bottom layer 12. The air channel 23 is connected with the air tube 40. When the measurement is completed, the air in the air bag 10 may be released through the air channel 23, thus significantly increasing the speed of deflation. Likewise, when performing blood pressure measurement, external air flow through the air tube 40 and the air channel 23 to inflate the air bag 10, thus increasing the speed of inflation. The arcuate configuration at the bottom surface 21 of the retaining plate 20 effectively enhances the speed and precision of blood pressure measurement. Meanwhile, when the sphygmomanometer cuff is fastened around the arm, the air gap 22 formed between the arcuate surface 21 and the arm facilitates skin respiration and enhances comfort of measurement.

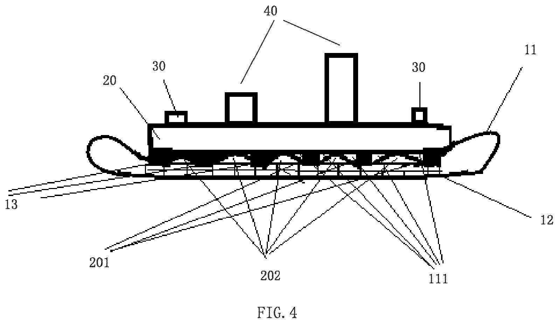

Referring to FIG. 4. A plurality of securing feet 201 (for example, a plurality of tiny posts) is formed intermittently on a bottom of the retaining plate 20, and contact the outer surface of the top layer 11 of the air bag 10. A plurality of interspaces 111 are formed between any two of the securing feet 201 at the bottom of the retaining plate 20 and the outer surface of the top layer 11.

When the sphygmomanometer cuff is worn on the arm for a long time, the retaining plate 20 abuts against the air bag 10, and the plurality of securing feet 201 contacts the outer surface of the top layer 11. When the air bag 10 is inflated, the securing feet 201 press against the top layer 11, and portions of the top layer 11, the bottom layer 12, and the intermediate layer 13 corresponding to the securing feet 201 are compressed together in the air bag 10. The plurality of interspaces 111 formed between the securing feet 201 depressurize the top layer 11, the bottom layer 12, and the intermediate layer 13, and internal pressure of the air bag 10 allows a plurality of air passages 202 to form at the interspaces 111. As the plurality of air passages 202 are connected to the air tube 40, fast inflation and deflation of the air bag 10 is achieved.

When the measurement is completed, the air bag 10 may be released through the plurality of air passages 202, thus increasing the speed of deflation. Likewise, when measuring blood pressure, the air passages 202 forms in the sealed cavity 101 during inflation, thus facilitating external air entry and enhance the speed of inflation. Thus, the air passages 202 may increase measurement speed and improve comfort of the measurement.

In addition, a wearable sphygmomanometer is provided in one embodiment. The wearable sphygmomanometer includes a sphygmomanometer main body and a sphygmomanometer cuff. The fastener 30 secures the sphygmomanometer main body to the upper surface of the retaining plate 20.

In addition, a method for using the wearable sphygmomanometer according to the aforementioned embodiments is provided. The method includes the following steps.

Providing the wearable sphygmomanometer cuff.

Wrapping the sphygmomanometer cuff around the arm of the user.

When the user wears the wearable sphygmomanometer around the arm for a long time, the air bag of the sphygmomanometer cuff abuts against the arm and the top layer and the bottom layer of the air bag are disposed adjacent to each other. The rough surface of the intermediate layer allows the plurality of gaps to form at the contact area between the sphygmomanometer cuff and the arm so as to improve skin respiration.

When the wearable sphygmomanometer performs blood pressure measurement, external air flows through the air tube to inflate the sealed cavity of the air bag. The rough surface of the intermediate layer prevents the top layer and the bottom layer from adhering to each other, so as to expedite inflation of the sealed cavity of the air bag.

When the measurement is completed, the air in the sealed cavity is released through the air tube. The rough surface of the intermediate layer prevents the top layer and the bottom layer from adhering to each other, so as to expedite deflation of the sealed cavity of the air bag.

When the sphygmomanometer cuff is worn on the arm of the user for a long time, the retaining plate abuts against the air bag, and the top layer and the bottom layer are disposed adjacent to each other. The arcuate surface of the retaining plate allows an air channel to form between the top layer and the bottom layer. The air channel is connected with the air tube for facilitating inflation and deflation of the air bag.

When the sphygmomanometer cuff is worn on the arm of the user for a long time, the retaining plate abuts against the air bag, and the plurality of interspaces formed between the securing feet allows the plurality of air passages to form in the sealed cavity when the air bag is inflated. The air passages are connected with the air tube, so that inflation and deflation of the air bag is facilitated.

According to the aforementioned embodiments, the sphygmomanometer cuff, the sphygmomanometer using the same, and the method for using the same have the following advantages.

First, the fabric intermediate layer disposed between the top layer and the bottom layer of the air bag can prevent adhesion between the top layer and bottom layer and thus facilitate fast inflation and deflation.

Second, when the sphygmomanometer cuff is worn on the arm for a long time, the intermediate layer also allows the gaps to form between the sphygmomanometer cuff and the arm so that skin respiration and thus comfort of blood pressure measurement is enhanced.

Third, when the sphygmomanometer cuff is worn on the arm for a long time, an air channel is defined in the top layer and the bottom layer of the air bag corresponding to the arcuate surface. An air channel is defined in the arcuate surface of the retaining plate between the top layer and the bottom layer and is connected with the air tube. When the measurement is completed, air in the air bag can be quickly deflated through the air channel. The speed of deflation of the air bag is improved. Similarly, when the sphygmomanometer starts to measure blood pressure and the air bag starts to be inflated. Outer air from the air tube can fill the air bag quickly through the air channel. The speed of inflation of the air bag is improved. Thus, arcuate surface of the retaining plate can improve the measurement speed and measurement precision. In addition, when the sphygmomanometer cuff presses the arm tightly, an air gap is defined between the arcuate surface and the arm, which facilitates skin respiration.

Finally, when the sphygmomanometer cuff is worn on the arm of the user for a long time, the retaining plate abuts against the air bag, the plurality of securing feet abuts against the top layer and the bottom layer of the air bag, the top layer and the bottom layer of the air bag are attached together. A plurality of intermittent air passages is defined in the top layer and the bottom layer corresponding to gaps of the plurality of securing feet, and the air passages are connected with the air tube, so as to fast inflate or deflate the air bag through the plurality of intermittent air passages between the top layer and the bottom layer of the air bag, when the air bag is inflated or deflated. In addition, when the sphygmomanometer cuff is pressured, the air bag presses the arm tightly. The plurality of gaps is defined between the plurality of securing feet and the arm, therefore facilitating skin respiration.

It is also to be understood, however, that even though numerous characteristics and advantages have been set forth in the foregoing description of the embodiments, together with details of the structures and functions of the embodiments, the disclosure is illustrative only, and changes may be made in detail, especially in matters of shape, size, and arrangement of parts within the principles of the disclosure to the full extent indicated by the broad general meaning of the terms in which the appended claims are expressed.

## Claims

1. A sphygmomanometer cuff for a wearable sphygmomanometer comprising a cuff body for wrapping around a predetermined portion of a body, the cuff body comprising an air bag (10), a retaining plate (20) and an air tube (40); the air bag (10) comprising a top layer (11) and a bottom layer (12), a sealed cavity (101) being defined between the top layer (11) and the bottom layer (12); the retaining plate (20) attached to an outer surface of the top layer (11) of the air bag (10); the air tube (40) extending through the retaining plate (20) and connecting with the sealed cavity (101) of the air bag (10), wherein an intermediate layer (13) is disposed between the top layer (11) and the bottom layer (12) and separates the top layer (11) and the bottom layer (12), and the intermediate layer (13) has a rough surface; **characterized in that**
the retaining plate (20) is suitable for securing a sphygmomanometer to the cuff body; and
a bottom surface (21) of the retaining plate (20) is arcuate, the bottom surface (21) recesses toward a top surface of the retaining plate (20), a plurality of securing feet (201) are formed intermittently on a bottom of the retaining plate (20) and intermittently contact the outer surface of the top layer (11) of the air bag (10), and a plurality of interspaces (111) are formed between any two of the securing feet (201) at the bottom of the retaining plate (20) and the outer surface of the top layer (11).

2. The sphygmomanometer cuff of claim 1, wherein the intermediate layer (13) is attached to an inner surface of the bottom layer (12), or an inner surface of the top layer (11).

3. A wearable sphygmomanometer, **characterized in** comprising the sphygmomanometer cuff of one of claims 1-2 and a sphygmomanometer main body, wherein the sphygmomanometer main body is attached to a top surface of the retaining plate (20).

4. A method for using a wearable sphygmomanometer, **characterized in** comprising:
providing a wearable sphygmomanometer of claim 3; and
wrapping the sphygmomanometer cuff around the predetermined portion of the body,
wherein when the wearable sphygmomanometer is worn on an arm for a long time, the air bag (10) of the sphygmomanometer cuff abuts against the arm, the top layer (11) and the bottom layer (12) of the air bag (10) are disposed adjacent to each other, and the rough surface of the intermediate layer of the air bag allows a plurality of gaps to form at a contact area between the cuff body and the predetermined portion of the body, so as to improve skin respiration,
when the wearable sphygmomanometer performs a measurement of blood pressure, external air flows through the air tube (40) to inflate the sealed cavity (101) of the air bag (10), and the rough surface of the intermediate layer (13) prevents the top layer (11) and the bottom layer (12) from adhering to each other, so as to expedite inflation of the sealed cavity (101) of the air bag (10), and
when the wearable sphygmomanometer completes the measurement, air in the sealed cavity (101) of the air bag is released through the air tube (40), and the rough surface of the intermediate layer (13) prevents the top layer (11) and the bottom layer (12) from adhering to each other, so as to expedite deflation of the sealed cavity (101) of the air bag (10).

5. The method of claim 4, wherein when the wearable sphygmomanometer is worn on an arm for a long time, the retaining plate (20) abuts against the air bag (10), the top layer (11) and the bottom layer (12) of the air bag (10) are disposed adjacent to each other, and an air channel (23) is defined in the arcuate surface of the retaining plate (20) between the top layer (11) and the bottom layer (12) and is connected with the air tube (40), so as to fast inflate or deflate the air bag (10).

6. The method of claim 4, wherein when the wearable sphygmomanometer is worn on an arm for a long time, the retaining plate (20) abuts against the air bag (10); when the air bag (10) is inflated, the securing feet (201) press against the top layer (11), and portions of the top layer (11), the bottom layer (12), and the intermediate layer (13) corresponding to the securing feet (201) are compressed together in the air bag (10), so that the top layer (11) and the bottom layer (12) of the air bag (10) abut against the plurality of securing feet to form a plurality of intermittent air passages (202) in the sealed cavity (101), and the air passages (202) are connected with the air tube (40), so as to fast inflate or deflate the air bag (10).

## Patentansprüche

1. Sphygmomanometer-Manschette für ein tragbares Sphygmomanometer, umfassend einen Manschettenkörper zum Wickeln um einen vorbestimmten Abschnitt eines Körpers, wobei der Manschettenkörper ein Luftkissen (10), eine Halteplatte (20) und ein Luftrohr (40) umfasst; wobei das Luftkissen (10) eine obere Schicht (11) und eine untere Schicht (12) umfasst, ein abgedichteter Hohlraum (101) zwischen der oberen Schicht (11) und der unteren Schicht (12) definiert ist; die Halteplatte (20) an einer Außenoberfläche der oberen Schicht (11) des Luftkissens (10) angebracht ist; sich das Luftrohr (40) durch die Halteplatte (20) hindurch erstreckt, und mit dem abgedichteten Hohlraum (101) des Luftkissens (10) verbunden ist, wobei eine Zwischenschicht (13) zwischen der oberen Schicht (11) und der unteren Schicht (12) angeordnet ist, und die obere Schicht (11) und die untere Schicht (12) trennt, und die Zwischenschicht (13) eine raue Oberfläche aufweist;
**dadurch gekennzeichnet, dass**
sich die Halteplatte (20) zum Befestigen eines Sphygmomanometers an dem Manschettenkörper eignet; und eine untere Oberfläche (21) der Halteplatte (20) bogenförmig ist, sich die untere Oberfläche (21) zu einer oberen Oberfläche der Halteplatte (20) vertieft, eine Vielzahl von Befestigungsfüßen (201) intermittierend an einem Boden der Halteplatte (20) gebildet sind, und intermittierend die Außenoberfläche der oberen Schicht (11) des Luftkissens (10) berühren, und eine Vielzahl von Zwischenräumen (111) zwischen jeweils zwei der Befestigungsfüße (201) am Boden der Halteplatte (20) und der Außenoberfläche der oberen Schicht (11) gebildet werden.

2. Sphygmomanometer-Manschette nach Anspruch 1, wobei die Zwischenschicht (13) an einer Innenoberfläche der unteren Schicht (12) oder einer Innenoberfläche der oberen Schicht (11) angebracht ist.

3. Tragbares Sphygmomanometer, **dadurch gekennzeichnet, dass** es die Sphygmomanometer-Manschette nach einem der Ansprüche 1 bis 2 und einen Sphygmomanometer-Hauptkörper umfasst, wobei der Sphygmomanometer-Hauptkörper an einer oberen Oberfläche der Halteplatte (20) angebracht ist.

4. Verfahren zum Verwenden eines tragbaren Sphygmomanometers, **dadurch gekennzeichnet, dass** es umfasst:
Bereitstellen eines tragbaren Sphygmomanometers nach Anspruch 3; und
Wickeln der Sphygmomanometer-Manschette um einen vorbestimmten Abschnitt des Körpers,
wobei, wenn das tragbare Sphygmomanometer lange an einem Arm getragen wird, das Luftkissen (10) der Sphygmomanometer-Manschette an dem Arm anliegt, die obere Schicht (11) und die untere Schicht (12) des Luftkissens (10) aneinander angrenzend angeordnet sind, und es die raue Oberfläche der Zwischenschicht des Luftkissens ermöglicht, dass sich eine Vielzahl von Lücken in einem Berührungsbereich zwischen dem Manschettenkörper und dem vorbestimmten Abschnitt des Körpers bilden, um die Hautatmung zu verbessern,
wenn das tragbare Sphygmomanometer eine Messung des Blutdrucks durchführt, Außenluft durch das Luftrohr (40) strömt, um den abgedichteten Hohlraum (101) des Luftkissens (10) aufzublasen, und die raue Oberfläche der Zwischenschicht (13) verhindert, dass die obere Schicht (11) und die untere Schicht (12) aneinander anhaften, um das Aufblasen des abgedichteten Hohlraums (101) des Luftkissens (10) zu beschleunigen, und
wenn das tragbare Sphygmomanometer die Messung beendet, Luft in dem abgedichteten Hohlraum (101) des Luftkissens durch das Luftrohr (40) hindurch abgelassen wird und die raue Oberfläche der Zwischenschicht (13) verhindert, dass die obere Schicht (11) und die untere Schicht (12) aneinander anhaften, um das Entleeren des abgedichteten Hohlraums (101) des Luftkissens (10) zu beschleunigen.

5. Verfahren nach Anspruch 4, wobei, wenn das tragbare Sphygmomanometer lange an einem Arm getragen wird, die Halteplatte (20) an dem Luftkissen (10) anliegt, die obere Schicht (11) und die untere Schicht (12) des Luftkissens (10) aneinander angrenzend angeordnet sind, und ein Luftkanal (23) an der bogenförmigen Oberfläche der Halteplatte (20) zwischen der oberen Schicht (11) und der unteren Schicht (12) definiert wird, und mit dem Luftrohr (40) verbunden ist, um das Luftkissen (10) rasch aufzublasen oder zu entleeren.

6. Verfahren nach Anspruch 4, wobei, wenn das tragbare Sphygmomanometer lange an einem Arm getragen wird, die Halteplatte (20) an dem Luftkissen (10) anliegt; wenn das Luftkissen (10) aufgeblasen ist, die Befestigungsfüße (201) gegen die obere Schicht (11) drücken, und Abschnitte der oberen Schicht (11), der unteren Schicht (12) und der Zwischenschicht (13), die den Befestigungsfüßen (201) entsprechen, gemeinsam in dem Luftkissen (10) zusammengedrückt werden, sodass die obere Schicht (11) und die untere Schicht (12) des Luftkissens (10) an der Vielzahl von Befestigungsfüßen anliegen, um eine Vielzahl von intermittierenden Luftdurchgängen (202) in dem abgedichteten Hohlraum (101) zu bilden, und die Luftdurchgänge (202) mit dem Luftrohr (40) verbunden sind, um das Luftkissen (10) rasch aufzublasen oder zu entleeren.

## Revendications

1. Brassard de sphygmomanomètre pour un sphygmomanomètre portable comprenant un corps de brassard pour s'enrouler autour d'une partie prédéterminée d'un corps, le corps de brassard comprenant un coussin d'air (10), une plaque de retenue (20) et un tube d'air (40) ; le coussin d'air (10) comprenant une couche supérieure (11) et une couche de fond (12), une cavité scellée (101) étant définie entre la couche supérieure (11) et la couche de fond (12) ; la plaque de retenue (20) attachée à une surface extérieure de la couche supérieure (11) du coussin d'air (10) ; le tube d'air (40) s'étendant à travers la plaque de retenue (20) et étant relié à la cavité scellée (101) du coussin d'air (10), dans lequel une couche intermédiaire (13) est disposée entre la couche supérieure (11) et la couche de fond (12) et sépare la couche supérieure (11) et la couche de fond (12), et la couche intermédiaire (13) présente une surface rugueuse ;
**caractérisé en ce que**
la plaque de retenue (20) est appropriée pour fixer un sphygmomanomètre au corps de brassard ; et
une surface de fond (21) de la plaque de retenue (20) est arquée, la surface de fond (21) s'enfonce vers une surface supérieure de la plaque de retenue (20), une pluralité de pieds de fixation (201) sont formés par intermittence sur un fond de la plaque de retenue (20) et sont en contact intermittent avec la surface extérieure de la couche supérieure (11) du coussin d'air (10), et une pluralité d'espaces intermédiaires (111) sont formés entre deux quelconques des pieds de fixation (201) au niveau du fond de la plaque de retenue (20) et la surface extérieure de la couche supérieure (11).

2. Brassard de sphygmomanomètre selon la revendication 1, dans lequel la couche intermédiaire (13) est attachée à une surface intérieure de la couche de fond (12), ou une surface intérieure de la couche supérieure (11).

3. Sphygmomanomètre portable, **caractérisé en ce qu'**il comprend le brassard de sphygmomanomètre selon l'une des revendications 1-2 et un corps principal de sphygmomanomètre, dans lequel le corps principal de sphygmomanomètre est attaché à une surface supérieure de la plaque de retenue (20).

4. Procédé pour utiliser un sphygmomanomètre portable, **caractérisé en ce qu'**il comprend :
la fourniture d'un sphygmomanomètre portable selon la revendication 3 ; et
l'enroulement du brassard de sphygmomanomètre autour de la partie prédéterminée du corps,
dans lequel, lorsque le sphygmomanomètre portable est porté sur un bras pendant longtemps, le coussin d'air (10) du brassard de sphygmomanomètre bute contre le bras, la couche supérieure (11) et la couche de fond (12) du coussin d'air (10) sont disposées de manière adjacente l'une à l'autre, et la surface rugueuse de la couche intermédiaire du coussin d'air permet à une pluralité d'espaces de se former au niveau d'une zone de contact entre le corps de brassard et la partie prédéterminée du corps, de manière à améliorer la respiration de la peau, lorsque le sphygmomanomètre portable effectue une mesure de pression artérielle, de l'air externe s'écoule à travers le tube d'air (40) pour gonfler la cavité scellée (101) du coussin d'air (10), et la surface rugueuse de la couche intermédiaire (13) empêche la couche supérieure (11) et la couche de fond (12) d'adhérer l'une à l'autre, de manière à accélérer le gonflage de la cavité scellée (101) du coussin d'air (10), et
lorsque le sphygmomanomètre portable termine la mesure, l'air dans la cavité scellée (101) du coussin d'air est libéré à travers le tube d'air (40), et la surface rugueuse de la couche intermédiaire (13) empêche la couche supérieure (11) et la couche de fond (12) d'adhérer l'une à l'autre, de manière à accélérer le dégonflage de la cavité scellée (101) du coussin d'air (10).

5. Procédé selon la revendication 4, dans lequel lorsque le sphygmomanomètre portable est porté sur un bras pendant longtemps, la plaque de retenue (20) bute contre le coussin d'air (10), la couche supérieure (11) et la couche de fond (12) du coussin d'air (10) sont disposées de manière adjacente l'une à l'autre, et un canal d'air (23) est défini dans la surface arquée de la plaque de retenue (20) entre la couche supérieure (11) et la couche de fond (12) et est relié au tube d'air (40), de manière à gonfler ou dégonfler rapidement le coussin d'air (10).

6. Procédé selon la revendication 4, dans lequel lorsque le sphygmomanomètre portable est porté sur un bras pendant longtemps, la plaque de retenue (20) bute contre le coussin d'air (10) ; lorsque le coussin d'air (10) est gonflé, les pieds de fixation (201) s'appuient contre la couche supérieure (11), et des parties de la couche supérieure (11), de la couche de fond (12) et de la couche intermédiaire (13) correspondant aux pieds de fixation (201) sont comprimées ensemble dans le coussin d'air (10), de telle sorte que la couche supérieure (11) et la couche de fond (12) du coussin d'air (10) butent contre la pluralité de pieds de fixation pour former une pluralité de passages d'air intermittents (202) dans la cavité scellée (101), et les passages d'air (202) sont reliés au tube d'air (40), de manière à gonfler ou dégonfler rapidement le coussin d'air (10).
